(19) Europäisches Patentamt
European Patent Office
Office européen des brevets

(11) **EP 2 677 445 A1**

(12) **EUROPEAN PATENT APPLICATION**

(43) Date of publication:
**25.12.2013 Bulletin 2013/52**

(51) Int Cl.:
**G06F 19/00** *(2011.01)*

(21) Application number: **12173060.0**

(22) Date of filing: **21.06.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **FUJITSU LIMITED**
**Kawasaki-shi,**
**Kanagawa 211-8588 (JP)**

(72) Inventors:
• **Hu, Bo**
  **Huntingdon, Cambridgeshire PE29 6JL (GB)**

• **Naseer, Aisha**
  **Hayes, Middlesex UB4 8FE (GB)**
• **Fukuda, Kenichi**
  **Hayes, Middlesex UB4 8FE (GB)**

(74) Representative: **Chapman, Patrick**
**Haseltine Lake LLP**
**5th Floor, Lincoln House**
**300 High Holborn**
**London WC1V 7JH (GB)**

(54) **Computer system, method and program to quantify the impact of a physical activity on a body**

(57)    A computer system (2) operable to quantify the impact of a physical activity on a body, the computer system comprising: a data model (4, 6) in which one or more physical activities are each decomposed into a plurality of fundamental actions which make up the physical activity and parts of the body and their relationships with one another are defined; an impact value generator (8) having access to the data model (4, 6) and an information source (10); wherein, for a specified physical activity from among the one or more physical activities, the impact value generator (8) is configured to generate an individual impact value for the impact of each fundamental action of the specified physical activity defined in the physical activity data model (4) on each body part defined in the data model (6); wherein each individual impact value is generated based on a correlation between the fundamental action and the body part in the information source (10).

FIG. 1

EP 2 677 445 A1

**Description**

The present invention relates to a computer system, method and program to quantify the impact of a physical activity on a body, particularly but not exclusively the human body.

As a result of the promotion of a healthy lifestyle, more and more people are participating in sports activities and/or low impact physical activities, such as power walking, Tai-Chi, and yoga.

Even though exercise is generally considered beneficial to the human body, sports injuries may occur. Such injuries are particularly common where a person makes a drastic change to their lifestyle, overdoes the activity, or does not adequately warm up. Profound knowledge of human anatomy, body dynamics and general medicine may be required in order to prevent such harmful consequences.

Hitherto, the knowledge of how physical activities can impact our body has been, to some extent, kept exclusively to specialists (for example, personal trainers, physicians, physiologists, physiotherapists, etc.) who have acquired such knowledge through years of professional education, training and experience. Access to such knowledge, therefore, becomes costly and, in many occasions, time consuming for ordinary individuals.

On the other hand, the Internet provides a comprehensive and easy-to-use knowledge repository which may be used to complement human specialists. Nevertheless, whilst the Internet makes such information available to users, its vast size makes it difficult for users to amalgamate and comprehend this information.

Accordingly, an ordinary user may have difficulty in assessing the impact of a physical activity on the body and in selecting an appropriate physical activity. Moreover, the impact of physical activities on the human body is normally expressed qualitatively, with no well-defined meaning of the terms used to describe the impact of a physical activity. Consequently, it is difficult to for users to compare information sources, let alone physical activities.

It is therefore desirable to harness the quantitative information available regarding the impact of physical activities and to therefrom derive a qualitative measure which can be readily understood by an ordinary user, thus allowing the user to easily compare physical activities and select an appropriate activity.

According to an aspect of the invention, there is provided a computer system operable to quantify the impact of a physical activity on a body. The computer system comprises: a data model in which one or more physical activities are decomposed into a plurality of fundamental actions which make up the physical activity and in which parts of the body and their relationships with one another are defined; an impact value generator having access to the data model and an information source. For a specified physical activity from among the one or more physical activities, the impact value generator is configured to generate an individual impact value for the impact of each fundamental action of the specified physical activity defined in the physical activity data model on each body part defined in the data model. Each individual impact value may be based on a correlation between the fundamental action and the body part in the information source.

The body may be the human body. Alternatively, the body may be that of other animals or mechanical structures, such as robots or robotic arms, which have a plurality of a movable component parts connected via joints.

The term "computer system" can refer to any computing system with memory, processing capability and inputs and outputs, for instance to a simple system with computing functionality in a single location (for example a server and possibly links to various devices) or to a more complex partially or fully distributed system or to a computer system provided as a "cloud" service.

When applied to the human body, the term "physical activity" is used primarily to refer to sports, but may also include other forms of non-competitive physical exercise. For example, physical activities may include flexibility exercises or low-impact exercises, such as yoga or Pilates. In addition, physical activities can include work-related and leisure time activities, and even routine housework or driving activities.

When referring to mechanical structures, the physical activity may be any form of movement exercised by the structure.

Although the body may contact an external medium during a physical activity, such as the ground when running or water when swimming, the term "impact" is used in a more abstract sense to refer to the effect of a physical activity on the human body, particularly joints and muscles.

The computer system can be used to study the impact on muscles by using a complete "muscle-activity" data model for query generation and impact propagation.

The term "data model" can refer to any model suitable for storing physical activity data or anatomic data. In particular, the data model may be a database, such as a relational database, in which the data is stored. The database may also include relationship information which links certain data items. The data model may be a semantic data model. The data model may be implemented as a separate physical activity data model in which the physical activities are decomposed into fundamental actions and a separate human (or otherwise) anatomical data model defining the body parts and their relationships. The data model forming the physical activity data model and the data model forming the human anatomic data model need not be the same type of data model.

The data model may define ontologies. Such ontologies are used to formally represent knowledge as a set of concepts within a domain, and the relationships between those concepts.

The physical activity may be decomposed to any appropriate level of granularity. Accordingly, the term "fundamental" does not require that the action is the most elementary of actions. In fact, the level of granularity must only be sufficient to define more than one fundamental action or move which is used in the physical activity. Nevertheless, it is desirable to decompose the physical activity to the lowest level of granularity, if possible.

The decomposition of the physical activity may have a number of levels. For example, tennis may be said to comprise the action of swinging the racquet. This action may be decomposed further into different types of shot, such as serving, forehand, backhand, etc. In this case, it is desirable to reach a fine granularity since the different type of shots may create different impacts on the body, particularly the shoulder in this example. The lowest level of actions (i.e. the finest granularity) reached in the decomposition may be regarded as defining the fundamental actions.

The number of fundamental actions may vary for different physical activities. However, this may arise from the complexity of the physical activity, rather than the granularity of decomposition used for each physical activity.

Similarly, the human anatomical data model may define the human body to any appropriate level of granularity. For example, the human anatomical data model may simply define the upper limb or may break down the upper limb into the arm, forearm, wrist and hand.

The correlation between the fundamental action and the body part in the information source may be based on whether the fundamental action and the body part are referred to in the same item (such as an individual web page) of the information source or in close proximity to one another. Accordingly, the term "correlation" is used to refer to the relationship or association between the fundamental action and the body part in the information source. That is, the individual impact values may be based on a co-occurrence of the fundamental action and the body part in the information source.

The impact value generator may be further configured to generate a combined impact value for the physical activity on each body part based on the individual impact values for that body part.

In other words, the impact value generator may combine the individual impact values for each fundamental action used in the physical activity to determine the overall effect of the physical activity on the body part.

The combined impact value may be generated based on a sum of the individual impact values. The individual impact values may be normalised before calculating the combined impact value.

The combined impact values for each body part may also be combined to give an overall impact value for the physical activity on the entire human body.

The (physical activity) data model may comprise a weight for each fundamental action and the impact value generator may be configured to apply each of the weights to the respective individual impact values when generating the combined impact value. For example, the weights may be based on the significance of the fundamental action (i.e. the proportion of time performing this action) in the physical activity.

The weights may be derived based on guidance from domain experts.

The weights may also be derived from the information source based on the correlation between the fundamental action and the physical activity. The correlation may provide an indication of the significance of a fundamental action in a physical activity.

If there is a strong correlation between the fundamental action and the physical activity, it may suggest that the fundamental action is heavily employed in the physical activity. Accordingly, a higher weight may be applied to such fundamental actions, whereas those fundamental actions which do not have a strong correlation with the physical activity may be assigned a lower weight.

The impact value generator may comprise a query generating module which is configured to create a plurality of search queries, each of the search queries being a combination of one of the fundamental actions and one of the body parts. In other words, the search query may be a word string defining a fundamental action and a body part.

The search queries may also include the physical activity itself. Accordingly, the search query may be a combination of a physical activity, a fundamental action and a body part. The search queries may also include non-fundamental actions of which the fundamental action is a subset. For example, in tennis, the fundamental action may be backhand which is a subset of the action swinging the racquet.

By including both the physical activity and fundamental action in the search query, it is possible to remove unrelated information which may use the same term but in a different context. For example, the fundamental action of jumping may feature is the sports of basketball and long jump. However, the action may impose very different impacts on the knees for the two sports.

The search queries may define every combination or permutation of the fundamental actions and the body parts.

The impact value generator may be configured to apply each of the search queries to the information source and may generate the individual impact values based on a number of results for each search query.

The search may be performed using the "and" (or conjunction) logical operator so that the results only yield pages which contain both the fundamental action (and physical activity) and the body part.

The impact value generator may apply each of the search queries using an Internet search engine, such as Google.

The individual impact values may be generated based on a distance measure. Such a distance measure is a semantic similarity measure which provides a correlation between the occurrence of the individual search terms. For example, a Normalised Google Distance (NGD) may be used. With this measure, if the individual search terms never occur together on the same web page, but do occur separately, their NGD is infinite. Conversely, if both terms always occur together, their NGD is zero. The NGD measure is not limited to searches performed via Google and may be applied analogously to other Internet search engines.

The information source may contain information regarding physical activities and their effects on the body. Such information is likely to be defined in qualitative terms and does not use well-defined semantics.

The information source may be the Internet (i.e. the entire collection of web pages available to the Internet search engine) or a subset of the Internet. The subset of the internet may be a specialist repository, such as PubMed.

The impact value generator may comprise an impact propagation module. For each body part, the impact propagation module may be configured to identify one or more adjacent body parts from the data model and to adjust the individual impact values based on the individual impact values of the adjacent body parts.

Each individual impact value for a specified body part and a specified fundamental action may be adjusted based on a correlation between the specified body part and the one or more adjacent body parts in the information source. For example, a linguistic propagation method may be used, such as latent semantic analysis.

Each individual impact value for a specified body part and a specified fundamental action may be adjusted based on the specified fundamental action and a connection or joint between the specified body part and the one or more adjacent body parts. That is, the propagation of the impact is based on the actual structure of the body (i.e. the human anatomy) and the effect of the fundamental action on the structure.

Each individual impact value may be adjusted based on the direction of impact of the specified fundamental action and a degree of freedom of the connection between the specified body part and each of the one or more adjacent body parts. Accordingly, the propagation from one body part to another can be assessed based on an angle between the direction of the fundamental action and the degree of freedom of the joint.

The individual impact values may be adjusted by calculating one or more propagated impact values (i.e. the impact propagated to the specified body part from each of the adjacent body parts) and adding the propagated impact values to the individual impact value to provide the adjusted individual impact value.

The computer system may further comprise a user interface. The user interface may display the combined and/or individual impact values to a user. The user interface may also allow the user to select a physical activity of interest from the physical activity data model. The user may select a plurality of physical activities for comparison. The user interface may also be used to select or tailor the information source. In addition, the user interface may allow the user to input personal information which can be used in calculating the impact values, particularly for the purposes of calculating the weights for the fundamental actions.

According to another aspect of the invention, there is provided a computer-implemented method operable to quantify the impact of a physical activity on a body. The method comprises: decomposing each of one or more physical activities into a plurality of fundamental actions which make up the physical activity; providing a data model defining parts of the body and their relationships with one another; and for a specified physical activity from among the one or more physical activities, generating an individual impact value for the impact of each fundamental action of the physical activity on each body part defined in the data model. Each individual impact value is generated based on a correlation between the fundamental action and the body part in an information source.

This method is equivalent to the system aspect described above and therefore features of the system as herein before described are applicable equally to the method aspect. The steps of the method may be carried out in another order and still achieve desirable results. Equally, the various components or modules described may be provided by the same hardware resource, and/or the various data models described may use the same memory resource.

According to another aspect of the invention, there is provided a computer program which when executed on a computer carries out the method of the preceding method aspect and/or which when downloaded onto a computer system causes it to become the computer system of the apparatus aspect as variously set out above.

The invention converts the qualitative information provided by the information source into a quantitative value.

Accordingly, the invention provides clear and well-defined semantics. In particular, a standardised output is provided for different physical activities which may be employed by a user to quickly and easily compare the impact values of different physical activities, thus allowing them to make an informed decision on which physical activities are suitable.

The impact values can be readily defined for the user without the need for specific guidance from a specialist, such as a doctor. The invention may also be used by such specialists to extend their knowledge base and to convey the otherwise qualitative information in a quantitative form which can be more easily understood by an ordinary user. The invention therefore may be regarded as a complementary service which is not intended to entirely replace trained specialists.

The impact values may be obtained on-demand and in a dynamic manner. That is, the search queries may be performed at the time of calculation. Consequently, the impact values are based on the latest, most up-to-date information and knowledge available on the Internet.

For a better understand of the present invention and to show more clearly how it may be carried into effect, reference will now be made, by way of example, to the accompanying drawings, in which:

Figure 1 is a schematic view of a computer system according to an embodiment of the present invention;

Figure 2 is an example of a portion of a human body ontology used by the computer system;

Figure 3 is an example of a portion of a physical activity ontology used by the computer system; and

Figure 4 is a more detailed view of the computer system of Figure 1.

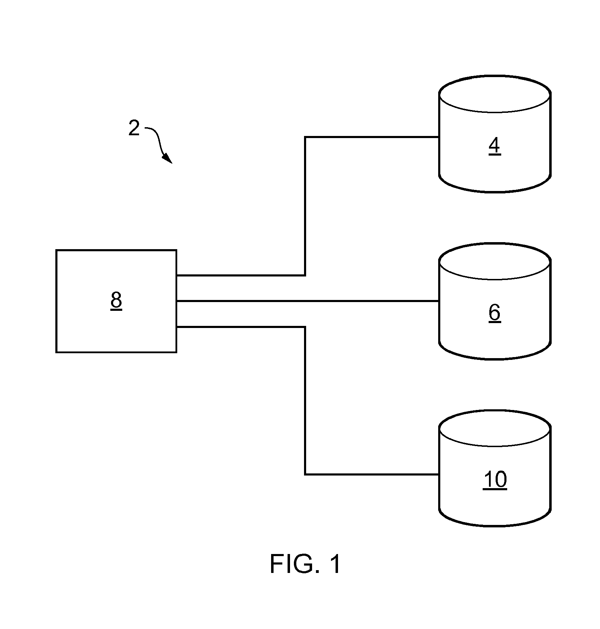

**Figure 1** shows a diagrammatic representation of a computer system 2 according to an embodiment of the invention which is operable to quantify the impact of a physical activity on the human body. The computer system 2 comprises a physical activity data model 4 and a human anatomic data model 6.

The physical activity data model 4 defines one or more physical activities. For example, the physical activity data model 4 may comprise a record for a plurality of physical activities, such as basketball, football (soccer), tennis, etc.

For each physical activity record the physical activity data model 4 contains a plurality of fundamental actions which make up the physical activity. In other words, the physical activity is decomposed into individual actions which are performed when partaking in the physical activity.

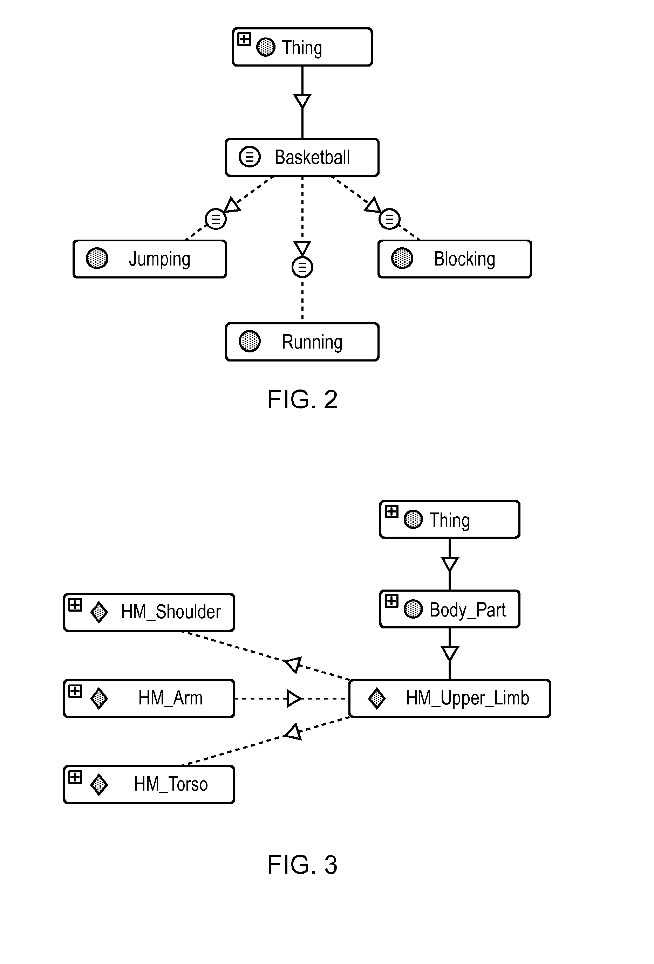

For example, as shown in the physical activity ontology of **Figure 2**, the physical activity basketball may be decomposed into the fundamental actions of running, jumping, blocking, etc. Similarly, football (soccer) may be decomposed into different types of tackle techniques, different styles of running and jumping, kicking, etc.

It is important to note that even though the same type of move might be employed in different physical activities, it may have a different significance for each activity, as will be described in more detail below.

These fundamental actions are recorded in the physical activity data model 4 for each physical activity. The physical activity data model 4 thus forms an ontology for the physical activities.

The human anatomic data model 6 defines a plurality of body parts forming the human anatomy. Further, the human anatomic data model 6 contains information regarding relationships between individual body parts. For example, the ontological relationships *"Part of"* and *"connect to"* may be used to define relationships between body parts. These relationships enable the propagation of impacts through the human body to be analysed, as will be described in more detail below.

For example, as shown in the human anatomy ontology of **Figure 3**, the upper limb is connected to the shoulder and torso, and comprises the arm, etc. (relationships with other body parts are not shown in Figure 3 for simplicity).

The computer system 2 further comprises an impact value generator 8. As shown in Figure 1, the impact value generator has access to the physical activity data model 4 and the human anatomic data model 6, and the data defined therein.

The impact value generator 8 also has access to information source 10. The information source 10 contains information regarding physical activities and their effects on the human body. Such information is likely to be defined in qualitative terms and does not use well-defined semantics. Consequently, it is difficult for an ordinary user to digest the information provided therein and to compare the information provided for different physical activities.

For a specified physical activity from among the one or more physical activities defined in the physical activity data model 4, the impact value generator 8 generates a plurality of individual impact values.

Each individual impact value corresponds to the impact of one of the fundamental actions of the specified physical activity, as defined in the physical activity data model 4, on one of the body parts, as defined in the human anatomic data model 6.

Each individual impact value is generated based on a correlation or relationship between the fundamental action and the body part in the information source.

Accordingly, the impact value generator produces a matrix of individual impact values for the specified physical activity. The matrix may contain rows of fundamental actions, columns of body parts (or vice versa), and cells containing the individual impact values.

Such a matrix may be provided for each physical activity defined in the physical activity data model 4. Alternatively, a matrix may be generated in response to a specific request from a user, as will be described in more detail below.

The individual impact values indicate to a user the level of impact on each body part as a result of the actions which make up the physical activity.

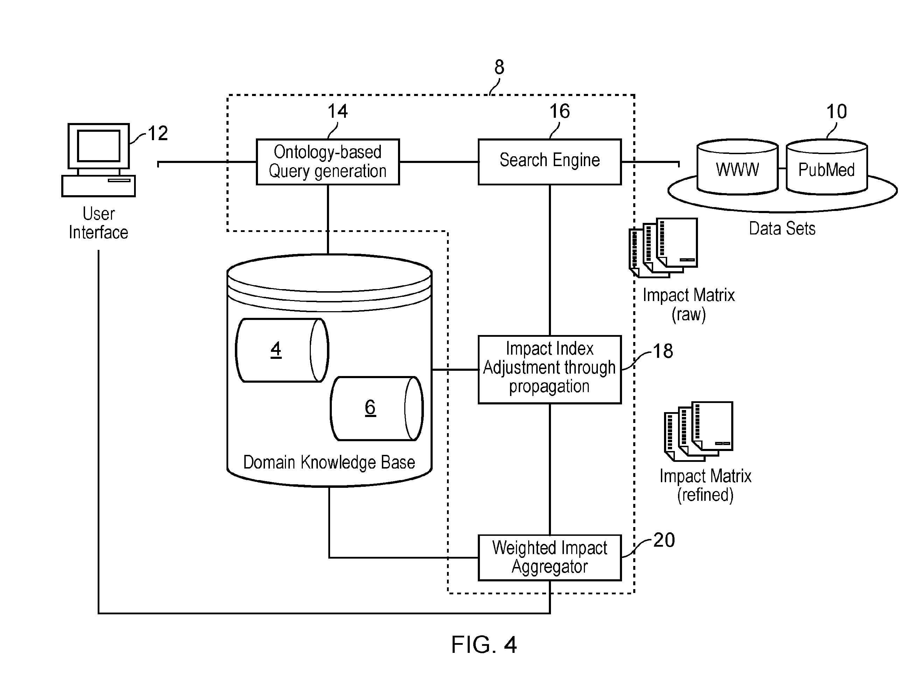

**Figure 4** shows a more detailed implementation of the computer system 2 which will now be described with reference to a corresponding method for quantifying the impact of a physical activity on the human body.

As described previously, the computer system 2 comprises the physical activity data model 4 and the human anatomic data model 6. As shown in Figure 4, the physical activity data model 4 and human anatomic data model 6 may be stored in the same storage medium as one another to form a domain knowledge base.

As shown in Figure 4, the information source 10 may be the Internet (WWW) or a specialist knowledge repository, such as PubMed. Such specialist knowledge repositories are generally Internet-based and thus may be regarded as a specific subset of the Internet. A specialist knowledge repository may be used to focus the impact value generation on medical information or other pertinent information, and to reduce extraneous noise created by unreliable or unrelated information sources.

The computer system may further comprise a user interface 12. The user interface 12 may be a graphical user interface which displays outputs from the impact value generator 8. The user interface 12 may also allow a user to input data to the impact value generator 8. For example, the user interface 12 may allow the user to specify one or more physical activities from those provided in the physical activity data model 4.

The user interface 12 may also allow the user to customise the operation of the impact value generator. For example, the user may wish to limit the information source to a specific knowledge repository. The user may also be able to input personal information via the user interface 12 so as to tailor the results to their body, as will be described in more detail below.

The impact value generator 8 comprises a query generating module 14. The query generating module 14 is configured to create a plurality of search queries based on the fundamental actions defined for the physical activity in the physical activity data model 4 and the body parts defined in the human anatomical data model 6.

The query generating module normalises the physical activities so as to give well formed search queries. For example, the fundamental actions of the physical activity basketball is normalised as the following: the concatenated search string is *running* n *basketball or blocking* ∩ *basketball or jumping* ∩ *basketball.*

The search queries may be generated using the following algorithm:

$$\textbf{function } \text{Query}\,(ph, Ph_o, Hm_o)\,\{$$

$$\textbf{for } \text{each sub move } mv_j \text{ of } ph$$

$$\text{concatenate the sub move } mv \text{ to } ph, \begin{cases} s_0 = ph \\ s_j = s_0 + \{mv\}_j \end{cases}$$

$$\textbf{for } \text{each body part } \beta \text{ in } Hm_o$$

$$q = s_j + \beta$$

$$q \rightarrow search\ engine$$

$$\textbf{end for}$$

$$\textbf{end for}$$

$$\textbf{end function}$$

where ph is the name of the physical activity, and $Ph_0$ and $Hm_0$ are the physical activity data model 4 and the human anatomic data model 6 respectively.

In other words, the algorithm concatenates each fundamental action (sub move) with the physical activity, and then adds each of the body parts in the human anatomic data model 6 in turn. The algorithm therefore provides search queries for every combination or permutation of fundamental actions and body parts.

The query generating module 14 then sends the generated search queries q to an Internet search engine 16, such as Google. The individual terms used in the search queries are linked using the "and" (or conjunction) logical operator. Consequently, the search only yields pages which contain the physical activity, the fundamental action and the body part.

As described previously, the information source 10 may be the entire Internet or a subset of the Internet, such as a knowledge repository. Accordingly, the search queries can be evaluated against the entire Internet (i.e. all of the pages available to the search engine) or a specialist knowledge repository. As described previously, a specialist knowledge repository may be used to focus the search on medical information or other pertinent information, and to reduce extraneous noise created by unreliable or unrelated information sources. The selection of the information source 10 may be performed by the user via the user interface 12. Alternatively, the information source 10 may be preset by the impact value generator 8.

For each search query, the impact value generator 8 calculates an individual impact value for the specific fundamental action and body part defined therein. The individual impact value is calculated based on the number of results found by the Internet search engine 16 for each search query.

For example, the Normalised Google Distance (NGD) may be used to calculate a numerical value defining the correlation between the individual search terms.

The Normalised Google Distance, which may be applied to other search engines, may be calculated using the following equation:

$$NGD\left(s_j, \beta_i\right) = \frac{\max\left(\log h\left(s_j\right), \log h\left(\beta_i\right)\right) - \log h\left(s_j, \beta_i\right)}{\log N - \min\left(\log h\left(s_j\right), \log h\left(\beta_i\right)\right)}$$

$$impact_{raw}\left(s_j, \beta_i\right) = correlation\left(s_j, \beta_i\right) = 1 - NGD\left(s_j, \beta_i\right)$$

where $\beta_i$ is the name of a human body part as defined in the human anatomic data model 6, $h()$ is the number of hits returned by the Internet search engine (for either the individual search term or the combination of search terms), and $N$ is the total number of pages indexed by the selected Internet search engine (currently about 47 billion from Google and around 12.5 billion from Bing).

Such a distance measure is a semantic similarity measure which provides a correlation between the occurrence of the individual search terms. With this measure, if the individual search terms never occur together on the same web page, but do occur separately, their NGD is infinite. Conversely, if both terms always occur together, their NGD is zero.

For example, the search query for the concatenation of the physical activity basketball and the fundamental action jumping returns $h(s_j)$ = 40,000,000 hits; the search query for knee returns $h(\beta_i)$ = 239,000,000 hits; and the search query for the combination of basketball, jumping and knee returns $h(s_j, \beta_i)$ = 13,100,000 hits. Therefore, this combination of fundamental action (and physical activity) and body part has an NGD of approximately 0.4.

The NGD may be converted into an individual impact value *(impact_{raw})* by subtracting the NGD from one. This gives the correlation between the fundamental action (and physical activity) and the body part.

This process may be repeated for every search query (i.e. every combination of a fundamental action and a body part). The size of j is equal to the cardinality of all the concepts in the physical activity data model 4 and the size of *i* is equal to the cardinality of all the concepts in the human anatomic data model 6.

The individual impact values may be arranged in a matrix having *j* rows of fundamental actions and *i* columns of body parts, or vice versa. The cells of the matrix define the individual impact values for each combination.

The raw data may provide useful information for the user by indicating the impact of each fundamental action of a physical activity on each body part. However, the individual impact values may be further refined to provide more accurate impact values. Further, the individual impact values for different body parts may be combined to provide a more readily understood value which can be easily compared for different physical activities.

Accordingly, the impact value generator 8 may further comprise an impact propagation module 16. The impact propagation module 16 is configured to refine the individual impact values by taking into account impacts which propagate to a body part from adjacent or neighbouring body parts.

For each body part, the impact propagation module 16 identifies one or more adjacent body parts. This is achieved using the human anatomic data model 6 and the relationships defined therein (i.e. the "connect_to" ontological relationship). For example, the impact propagation module 16 may identify that the knee is adjacent to the ankle and thus that impacts experienced by the ankle may propagate to the knee.

For each individual impact value, the impact propagation module 16 calculates one or more propagated impact values for each of the adjacent body parts.

The propagated impact values are based on the individual impact values for the adjacent body parts. That is, if the individual impact value of an adjacent body part indicates that the adjacent body part does not experience any impact, the propagated impact value for that adjacent body part will be zero. Consequently, no impact will propagate from the adjacent body part to the specified body part. Therefore, the individual impact value for the specified body part will not be adjusted by the propagated impact value.

Each propagated impact value for a specified body part and a specified fundamental action may be based on a correlation between the specified body part and the one or more adjacent body parts in the information source 10. For example, propagation may be performed using a form of linguistic propagation, such as latent semantic analysis. On the other hand, the propagated impact value may be based on the physical anatomy of the human body and the nature of the fundamental action.

The human anatomic data model 6 may further define the type of connection between adjacent body parts. In particular, a simplified human body Degrees of Freedom (DoF) scheme may be defined. The simplified DoF model may essentially divide joints between body parts into two types: hinge joints which have primarily one degree of freedom; and ball-socket joints which have three degrees of freedom. For example, the elbow and knee may be classified as hinge joints and the shoulder, hip, ankle, wrist, neck and waist may be classified as ball-socked joints.

The DoF model may also define the joint direction. For example, the shoulder joins the torso in a direction orthogonal to the torso. Again, this information is provided in the human anatomic data model 6.

The DoF model may be used by the impact propagation module 16 to assess how impacts will propagate through the human body. In order to assess impact propagation, the impact propagation module 16 also requires information regarding the nature of the fundamental action. For example, in basketball, the fundamental action of jumping suggests that the impact will propagate along the vertical (z) axis, whereas the fundamental action of blocking may incur impact more along the horizontal (x, y) axes. This information is provided in the physical activity data model 4.

The impact propagation module 16 may adjust the individual impact values to allow for propagation from adjacent body parts using the following equation:

$$impact_{prop}(s_j, \beta_i) = impact_{raw}(s_j, \beta_i) + \sum \cos \theta_k \cdot impact_{raw}(s_j, \beta_k)$$

where $\beta_k$ is an adjacent body part of the specified body part $\beta_i$ defined by the human anatomic data model 6 (with the sum being performed over all such adjacent body parts); and $\theta_k$ is the angle between the direction of the fundamental action defined in the physical activity data model 4 and the direction of impact propagation along the human body structure.

For instance, a horizontal blocking action will impact on the ankle which is adjacent to the knee. However, the impact will not propagate from the ankle to the knee since the impact direction is orthogonal to the normal DoF of the knee joint, and thus $\cos \theta_k * 0$.

The information defining the DoF model and the information regarding the type of fundamental action may be gained from domain experts when creating the physical activity data model 4 and the human anatomic data model 6.

Both methods of propagation adjustment may be used together. For example, linguistic propagation may be applied initially to ensure well-founded semantics prior to performing an anatomical-based propagation technique.

The individual impact values are adjusted based on the propagation from adjacent body parts. This information is compiled in a refined matrix of individual (propagation-adjusted) impact values for the specified physical activity. As described previously, the matrix may contain rows of fundamental actions, columns of body parts (or vice versa), and cells containing the individual (propagation-adjusted) impact values.

As described above, the individual impact values for different body parts may be combined to provide a more readily understood value which can be easily compared for different physical activities.

Accordingly, the impact value generator 8 comprises an impact value combining module 20 (weighted impact aggregator), as shown in Figure 4.

The combined impact value may be based on the individual impact values after they have been adjusted to take into account the propagation of impacts from adjacent body parts.

The impact value combining module 20 first normalises each of the individual impact values to provide values between 0 and 1. This is achieved using the following equation, where *i* is the number of concepts in the human anatomic data model 6:

$$impact_{norm}(mv_j, \beta_i) = impact_{norm}(s_j, \beta_i) = \frac{impact_{prop}(s_j, \beta_i)^2}{\sum_i impact_{prop}(s_j, \beta_i)^2}$$

The combined impact value of the physical activity ph on human body part $\beta_i$ is then calculated using the following equation:

$$impact(ph, \beta_i) = \sum_j \omega_j \cdot impact_{norm}(mv_j, \beta_i)$$

where $\omega_j$ is a weight attributed to each fundamental action $mv_j$ based on how much the fundamental action contributes to the physical activity ph.

The weights attributed to each fundamental action may be manually specified by domain experts against each individual. This approach may be desirable since it allows better personalisation of the impact values. In particular, the impact of a physical activity may vary depending on the physical strength of an individual, how much the individual is exposed to and accustomed to a particular type of activity and his/her biological profile. This information may be provided by the individual's doctor or qualified personal trainer. Such a personal profile can be composed once and repeatedly used when deriving the impact values for different physical activities. The accuracy of the profile may be subject to periodical review by the human experts or consultants. This information may be inputted into the impact value combining module 20 using the user interface 12.

The manual approach described above can be complemented by an Internet search engine-based weighting scheme. This may provide an indication of the significance of a fundamental action in a physical activity based on the correlation of the fundamental action and the physical activity. For example, an NGD (and correlation) value may be defined for the fundamental action in the same way as described above, based on the number of hits for the individual search terms and the number of hits for the combined search query (i.e. the fundamental action and the physical activity).

If there is a strong correlation between the fundamental action and the physical activity, it suggests that the fundamental action is heavily employed in the physical activity. Accordingly, a higher weight is applied to such fundamental actions, whereas those fundamental actions which do not have a strong correlation with the physical activity are assigned a lower weight.

As described above, the computer system 2 provides an impact value for the impact of a physical activity on a particular body part. The computer system 2 therefore converts the qualitative information provided by the Internet into a quantitative value. The impact values for a physical activity (i.e. $impact(ph, \beta_i)$) may be displayed to the user via the user interface 12.

The impact values can be delivered to the user as plain text or as a sorted list ranked in order of impact value (with the body parts experiencing the highest impact located near the top of the list, for example). Graphical presentation can also be used to overlay the results on a body figure. For example, colour coding may be used to represent the significance or magnitude of impacts on different parts of the body, thus forming a contour map of impact values.

The actual choice of delivering mechanism depends on the application and on user preference. For professional users (e.g. personal trainer, physiotherapist, etc.) an interactive method might be desirable, through which the users can provide domain heuristics to assist the refinement of the impact values.

If required, the matrices comprising the raw individual impact values and the individual propagation-adjusted impact values may also be displayed to the user via the user interface 12.

The computer system 2 and method described herein are able to quantify the impact of a physical activity on the human body. Accordingly, the computer system 2 and method provide clear and well-defined semantics. In other words, the computer system 2 and method provide a standardised output for different physical activities. This forms a Human Body Impact Index (HBI[2]). This measure may be employed by a user to quickly and easily compare the impact values of different physical activities, thus allowing the user to make an informed decision on which physical activities to partake in.

The impact values can be readily defined for the user without the need for specific guidance from a specialist, such as a doctor. However, the computer system 2 and method may also be used by such specialists to extend their knowledge base and to convey the otherwise qualitative information to an ordinary user in a quantitative form which can be more easily understood by the user. The invention may therefore be regarded as a complementary service which is not intended to entirely replace trained specialists.

The impact values may be obtained on-demand and in a dynamic manner. That is, the search queries may be performed at the time of calculation. Consequently, the impact values are based on the latest, most up-to-date information and knowledge available on the Internet.

Although the invention has been described with reference to quantifying the impact of a physical activity on the human body, it may be applied analogously to other animals. Moreover, the invention may be used to quantify the impact of physical activities on mechanical structures, such as robots or robotic arms, which have a plurality of movable component parts connected via joints.

The invention may also be used to study the impact on muscles by using a "muscle-activity" data model for query generation and impact propagation.

Although the physical activity data model 4 and the human anatomic data model 6 have been described as separate entities they may in fact be incorporated into a single data model.

## Claims

1. A computer system operable to quantify the impact of a physical activity on a body, the computer system comprising:

   a data model in which one or more physical activities are each decomposed into a plurality of fundamental actions which make up the physical activity and in which parts of the body and their relationships with one another are defined;
   an impact value generator having access to the data model and an information source;
   wherein, for a specified physical activity from among the one or more physical activities, the impact value generator is configured to generate an individual impact value for the impact of each fundamental action of the specified physical activity defined in the physical activity data model on each body part defined in the data model;
   wherein each individual impact value is generated based on a correlation between the fundamental action and the body part in the information source.

2. A computer system as claimed in claim 1, wherein the impact value generator is further configured to generate a combined impact value for the physical activity on each body part based on the individual impact values for that body part.

3. A computer system as claimed in claim 2, wherein the combined impact value is generated based on a sum of the individual impact values.

4. A computer system as claimed in claim 2 or 3, wherein the physical activity data model comprises a weight for each fundamental action and the impact value generator is configured to apply each of the weights to the respective individual impact values when generating the combined impact value for the specified physical activity.

5. A computer system as claimed in any preceding claim, wherein the impact value generator comprises a query generating module which is configured to create a plurality of search queries, each of the search queries being a combination of one of the fundamental actions and one of the body parts.

6. A computer system as claimed in claim 5, wherein the search queries define every combination of the fundamental actions and the body parts.

7. A computer system as claimed in claim 5 or 6, wherein the impact value generator is configured to apply each of the search queries to the information source and to generate the individual impact values based on a number of results for each search query.

8. A computer system as claimed in claim 7, wherein the impact value generator applies each of the search queries using an Internet Search Engine.

9. A computer system as claimed in claim 7 or 8, wherein the individual impact values are generated based on a distance measure.

**10.** A computer system as claimed in any preceding claim, wherein the information source is the Internet or a subset of the Internet.

**11.** A computer system as claimed in any preceding claim, wherein the impact value generator comprises an impact propagation module which, for each body part, is configured to identify one or more adjacent body parts from the data model and to adjust the individual impact values based on the individual impact values of the adjacent body parts.

**12.** A computer system as claimed in claim 11, wherein each individual impact value for a specified body part and a specified fundamental action is adjusted based on a correlation between the specified body part and the one or more adjacent body parts in the information source.

**13.** A computer system as claimed in claim 11 or 12, wherein each individual impact value for a specified body part and a specified fundamental action is adjusted based on the specified fundamental action and a connection between the specified body part and the one or more adjacent body parts.

**14.** A computer system as claimed in claim 13, wherein each individual impact value is adjusted based on the direction of impact of the specified fundamental action and a degree of freedom of the connection between the specified body part and each of the one or more adjacent body parts.

**15.** A computer system as claimed in any preceding claim, wherein the body is the human body.

**16.** A computer-implemented method operable to quantify the impact of a physical activity on a body, the method comprising:

decomposing each of one or more physical activities into a plurality of fundamental actions which make up the physical activity;
providing a data model defining parts of the body and their relationships with one another; and
for a specified physical activity from among the one or more physical activities, generating an individual impact value for the impact of each fundamental action of the physical activity on each body part defined in the data model;
wherein each individual impact value is generated based on a correlation between the fundamental action and the body part in an information source.

**17.** A computer program which when executed on a computer carries out the method of the preceding method claim and/or which when downloaded onto a computer system causes it to become the computer system of any of the preceding apparatus claims.

FIG. 1

⊞ ◉ Thing

▽

⊜ Basketball

⊜ ◉ Jumping

⊜ ◉ Blocking

⊜ ◉ Running

## FIG. 2

⊞ ◉ Thing

▽

⊞ ◉ Body_Part

▽

⊞ ◈ HM_Shoulder

⊞ ◈ HM_Arm

◈ HM_Upper_Limb

⊞ ◈ HM_Torso

## FIG. 3

FIG. 4

EP 2 677 445 A1

**EP 2 677 445 A1**

| | Europäisches Patentamt |
| | European Patent Office |
| | Office européen des brevets |

# EUROPEAN SEARCH REPORT

**Application Number**

EP 12 17 3060

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/013631 A1 (UNIV TOKYO [JP]; NAKAMURA YOSHIHIKO [JP]; YAMANE KATSU [JP]; TAKAYA SA) 4 February 2010 (2010-02-04) * abstract * * figure 5 * ----- | 1-4, 10-17 | INV. G06F19/00 |
| X | WO 2009/099340 A1 (ANDERSON IAIN ALEXANDER [NZ]; GILMOUR ROBERT FARRER [NZ]; HUNTER PETER) 13 August 2009 (2009-08-13) * page 5, line 32 - page 12, line 26 * ----- | 1-4, 10-17 | |
| X | WO 2008/015565 A2 (AUCKLAND UNISERVICES LTD [NZ]; SMITH NICOLAS PETER [GB]; BUDGETT DAVID) 7 February 2008 (2008-02-07) * paragraph [0011] - paragraph [0023] * ----- | 1-4, 10-17 | |
| A | EP 1 782 733 A1 (UNIV TOKYO [JP]) 9 May 2007 (2007-05-09) * column 28, line 37 - column 32, line 21 * ----- | 1-4, 10-17 | TECHNICAL FIELDS SEARCHED (IPC) G06F |
| A | EP 0 520 099 A1 (SHUKYOHOJIN KONGO ZEN SOHONZAN [JP]) 30 December 1992 (1992-12-30) * column 28, line 37 - column 32, line 21 * ----- | 1,16,17 | |

~~The present search report has been drawn up for all claims~~

| Place of search | Date of completion of the search | Examiner |
|---|---|---|
| Munich | 13 September 2012 | Rinelli, Pietro |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**Application Number**

EP 12 17 3060

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-4, 9-17

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 12 17 3060

The Search Division considers that the present European patent application does not comply with the
requirements of unity of invention and relates to several inventions or groups of inventions, namely:

```
    1. claims: 1-4, 9-17

            Impact of a physical activity on a body
                        ---

    2. claims: 5-8

            Interrogation of an information source
                        ---
```

## ANNEX TO THE EUROPEAN SEARCH REPORT
## ON EUROPEAN PATENT APPLICATION NO.

EP 12 17 3060

This annex lists the patent family members relating to the patent documents cited in the above-mentioned European search report.
The members are as contained in the European Patent Office EDP file on
The European Patent Office is in no way liable for these particulars which are merely given for the purpose of information.

13-09-2012

| Patent document cited in search report | | Publication date | Patent family member(s) | | Publication date |
|---|---|---|---|---|---|
| WO 2010013631 | A1 | 04-02-2010 | JP 2010029340 A | | 12-02-2010 |
| | | | WO 2010013631 A1 | | 04-02-2010 |
| WO 2009099340 | A1 | 13-08-2009 | AU 2009210884 A1 | | 13-08-2009 |
| | | | CA 2713861 A1 | | 13-08-2009 |
| | | | EP 2252953 A1 | | 24-11-2010 |
| | | | US 2011112808 A1 | | 12-05-2011 |
| | | | WO 2009099340 A1 | | 13-08-2009 |
| WO 2008015565 | A2 | 07-02-2008 | GB 2454384 A | | 06-05-2009 |
| | | | US 2010106475 A1 | | 29-04-2010 |
| | | | WO 2008015565 A2 | | 07-02-2008 |
| EP 1782733 | A1 | 09-05-2007 | EP 1782733 A1 | | 09-05-2007 |
| | | | JP 4590640 B2 | | 01-12-2010 |
| | | | US 2007256494 A1 | | 08-11-2007 |
| | | | WO 2005122900 A1 | | 29-12-2005 |
| EP 0520099 | A1 | 30-12-1992 | NONE | | |

EPO FORM P0459

For more details about this annex : see Official Journal of the European Patent Office, No. 12/82